# EUROPEAN PATENT APPLICATION

(11) **EP 4 431 616 A1**
(43) Date of publication of application: **18.09.2024**
(21) Application number: 22892805.7
(22) Date of filing: 09.11.2022
(51) Int. Cl.: C12Q 1/686, C12N 15/09, C12Q 1/6876, G01N 33/50

(54) **DIAGNOSIS MARKER DETECTION METHOD AND DIAGNOSTIC KIT**

(30) Priority: 10.11.2021 JP 2021183638
(71) Applicant: Isan Bio Inc., Yokohama-shi, Kanagawa 227-0036 (JP)
(72) Inventor: MITSUNAGA Shigeki, Mishima-shi, Shizuoka 411-8540 (JP); INOUE Ituro, Mishima-shi, Shizuoka 411-8540 (JP)
(74) Representative: Symbiosis IP Limited
(86) International application number: PCT/JP2022/041683
(87) International publication number: WO 2023/085307

(57) **Abstract**

A diagnosis maker detection method comprising a disease-related gene from circulating nucleic acids (CAN) that is in peripheral blood isolated from a subject.

## Description

### Technical Field

The present invention relates to a diagnosis marker detection method and a diagnostic kit.

### Background Art

Sporadic Alzheimer's disease (SAD) is a major cause of dementia. This is characterized by accumulation of amyloid β (hereinafter, also referred to as Aβ) in plaque and accumulation of abnormally phosphorylated tau in neurofibrillary tangle. These accumulations cause damage to neurons, but pathogenetic mechanisms are not fully understood.

Further, it has been reported on the basis of Aβ positron-emission tomography (amyloid PET) that accumulation of Aβ starts about 20 years before the onset of dementia. This long-term latent accumulation makes difficult early clinical diagnosis of dementia and mild cognitive impairment (MCI) which later develops into dementia.

Accumulation of Aβ is examined by amyloid PET, which is radiative, invasive, and costly, and can be utilized only in limited facilities. Aβ42, phosphorylated tau and total tau are main components of Aβ and are also measured in cerebral spinal fluid (CSF) as biomarkers for SAD, but collection of CSF is still invasive.

For this reason, research and development of blood biomarkers for early diagnosis and screening tests is being carried out. In addition to proteins/peptides measured in CSF, miRNA, long non-coding RNA (lncRNA), mRNA and circular RNA are being studied. The approaches of using these various blood RNAs as a biomarker generally involve combination of many kinds of RNA and are a little complicated.

Brain shrinkage is observed with age, with its degree being noticeable in SAD. The shrinkage of a lateral amygdala, a hippocampus, an entorhinal cortex and a parahippocampal cortex is observed even early after the onset. Studies are being also conducted on prediction of progress from NCI to SAD with an MRI image on the basis of a difference between the degrees of shrinkage in SAD and non-cognitive impairment (NCI). For example, in VSRAD, a region of interest (ROI) in a middle temporal structure such as an entorhinal cortex, a hippocampus or a lateral amygdala is analyzed, and shrinkage is more predominant in SAD patients.

On the other hand, a genome mosaic phenomenon from somatic variation exists in the brain and is observed in both SAD and NCI. The somatic variation includes a single nucleotide variant (SNV), a copy number variant (CNV), aneuploid, and retrotransposon activation. These genome mosaics are observed in the brain after death and are difficult to use as a biomarker.

Recently, existence of a genomic cDNA of an amyloid precursor protein (hereinafter, also referred to as APP) (APP gencDNA) which is one of brain mosaic phenomena and a transcript thereof in the brain after death have been reported.

The APP gencDNA exists in the form of being incorporated in genomic DNA and is characterized by having no intron and having an intra-exon junction. The APP gencDNA is specific to a neuron, and the number of Focis in DNA in situ hybridization (DISH) analysis is higher in SAD than in NCI. It has also been reported through the use of a mouse model of SAD that the number of DISH focis in APP gencDNA increases with age. The blood plasma nucleic acid level has been reported to increase more in SAD than in NCI. Since brain shrinkage is caused by necrosis/apoptosis, these reports indicate that APP gencDNA and a transcript thereof are released into blood plasma from a damaged neuron like other cfDNAs and cfRNAs (see, for example, Non-Patent Document 1).

Further, it has been recently reported through the use of retinal ganglion cells of mice that a NUMB endocytic adaptor protein gene (hereinafter, a NUMB gene) is involved in regulation of a tau protein level which is involved in the onset of Alzheimer's disease (see, for example, Non-Patent Document 2). It has been reported that a prokineticin 2 (hereinafter, a PROK 2 gene) is also involved in Alzheimer's disease (see, for example, Non-Patent Document 3). The NUMB gene and PROK 2 gene are expected to leak from damaged neurons like APP gencDNA.

### Citation List

### Non-Patent Documents

Non-Patent Document 1: Lee, M. H. et al. Somatic APP gene recombination in Alzheimer's disease and normal neurons. Nature 563, 639-645 (2018).
Non-Patent Document 2: Lacomme, M. et al. Numb regulates Tau levels and prevents neurodegeneration in tauopathy mouse models. Sci Adv. 2022 Oct 21;8(42): eabm4295. doi: 10.1126/sciadv.abm4295. Epub 2022 Oct 19
Non-Patent Document 3: Zuena, A.R. et al. Chemokines in Alzheimer's Disease: New Insights Into Prokineticins, Chemokine-Like Proteins. Front Pharmacol. 10,622 (2019).

### Summary of Invention

### Technical Problem

It is acknowledged that the amount of APP gencDNA is small because it is recombinantly formed in somatic cells as opposed to being formed in germline cells. Thus, the amount of APP gencDNA in blood plasma may be very small. However, since nucleic acids are easily amplified unlike proteins, it may be possible to detect APP gencDNA even in blood plasma if amplification can be performed. In addition to ease of detectability of nucleic acid in comparison with peptide, damage to neurons and release of related nucleic acids can be detected even before the onset of dementia. That is, it is indicated that if APP gencDNA in blood plasma can be detected, SAD can be early diagnosed. Alternatively, it is indicated that if damaged neurons and nucleic acids released from glial cells can be detected, SAD can be early diagnosed.

The present invention has been made in view of the above-described circumstances and provides a diagnosis marker detection method and a diagnostic kit which enable convenient, sensitive, rapid and noninvasive diagnosis of Alzheimer's disease.

### Solution to Problem

The present invention includes the following aspects.
[1] A diagnosis marker detection method comprising detecting a disease-related gene from circulating nucleic acids (CNAs) in peripheral blood isolated from a subject.
[2] The diagnosis marker detection method according to [1], wherein the disease is Alzheimer's disease.
[3] The diagnosis marker detection method according to [1] or [2], wherein the disease-related gene is an amyloid precursor protein (APP) gene.
[4] The diagnosis marker detection method according to any one of [1] to [3], wherein a site of connection between exons of the disease-related gene is detected.
[5] The diagnosis marker detection method according to [4], wherein the site of connection between exons of the disease-related gene is a site of connection between exon 7 and exon 9 of an APP gene.
[6] The diagnosis marker detection method according to [4], wherein the site of connection between exons of the disease-related gene is a site of connection between exon 14 and exon 16 of an APP gene.
[7] The diagnosis marker detection method according to any one of [1] to [3], wherein a site of homologous recombination between exons of the disease-related gene is detected.
[8] The diagnosis marker detection method according to [1] or [2], wherein the disease-related gene is a NUMB endocytic adaptor protein gene (NUMB gene) or a prokineticin 2 gene (PROK 2 gene).
[9] The diagnosis marker detection method according to [1] or [2], wherein the disease-related gene is a combination genes selected from the group consisting of an APP gene, a NUMB endocytic adaptor protein gene (NUMB gene) and a prokineticin 2 gene (PROK 2 gene).
[10] The diagnosis marker detection method according to any one of [1] to [9], wherein further, the number of copies of the detected disease-related gene is quantitated.
[11] The diagnosis marker detection method according to any one of [4] to [10], wherein the quantitation is performed by, using a reverse transcript of CNA purified from blood plasma, amplifying a region containing the site of connection or the site of homologous recombination between exons, the NUMB gene or the PROK 2 gene by singleplex PCR or multiplex PCR; analyzing a nucleotide sequence of an amplification product thereof with a next-generation sequencer (NGS); and counting the number of reads in the region containing the site of connection or the site of homologous recombination between exons, or the NUMB gene or the PROK 2 gene.
[12] The diagnosis marker detection method according to any one of [4] to [9], wherein the region containing the site of connection or the site of homologous recombination between exons, or the NUMB gene or the PROK 2 gene is quantitated by a singleplex or multiplex one-step real-time PCR (RT-PCR) method comprising a reverse transcription step.
[13] The diagnosis marker detection method according to [11] or [12], wherein standardization is performed by, during PCR of the region containing the site of connection or the site of homologous recombination between exons, or PCR of the NUMB gene or PCR of the PROK 2 gene, amplifying an internal standard in the same PCR tube; and comparing the number of reads in the internal standard with the number of reads in the site of connection or the site of homologous recombination between exons, or the NUMB gene or the PROK 2 gene; or standardization is performed by, during RT-qPCR, using a fluorescent dye-labeled probe specific to the site of connection or the site of homologous recombination between exons, or the NUMB gene or the PROK 2 gene, and an internal standard-specific fluorescent dye-labeled probe labeled with a fluorescent dye different from a fluorescent dye of the fluorescent dye-labeled probe specific to the site of connection or the site of homologous recombination between exons, or the NUMB gene or the PROK 2 gene; and comparing fluorescence intensities of the probes.
[14] The diagnosis marker detection method according to [13], wherein the internal standard contains exon 8 of an APP gene.
[15] The diagnosis marker detection method according to [14], wherein the internal standard contains an exon region of an HLA-DRA gene.
[16] A diagnostic kit for detecting a disease-related gene from circulating nucleic acids (CNAs) in peripheral blood isolated from a subject, the kit comprising
   a primer set for amplifying the disease-related gene, and/or a probe that binds to the disease-related gene or an amplification product thereof.
[17] The diagnostic kit according to [16], wherein the disease is Alzheimer's disease.
[18] The diagnostic kit according to [16] or [17], wherein the disease-related gene is at least one selected from the group consisting of an amyloid precursor protein (APP) gene, a NUMB endocytic adaptor protein gene (NUMB gene) and a PRPK 2 gene.
[19] The diagnostic kit according to any one of [16] to [18], wherein a site of connection, site of homologous recombination between exons or an exon of the disease-related gene is detected.

### Advantageous Effect of Invention

According to the present invention, it is possible to provide a diagnosis marker detection method and a diagnostic kit which enable convenient, sensitive, rapid and noninvasive diagnosis of Alzheimer's disease.

### Brief Description of Drawings

[FIG. 1] Figure 1 shows structures of an APP gene and mRNA.
[FIG. 2] Figure 2 is a schematic diagram of the present invention. Four published NGS data (sequence, read, archive and SRA) were analyzed with a computer using prepared probe sequences. In the present invention, four SRAs were analyzed using a probe sequence. Further, circulating nucleic acids extracted 1 to 1.2 mL of a blood plasma sample were analyzed with an Illumina sequence.

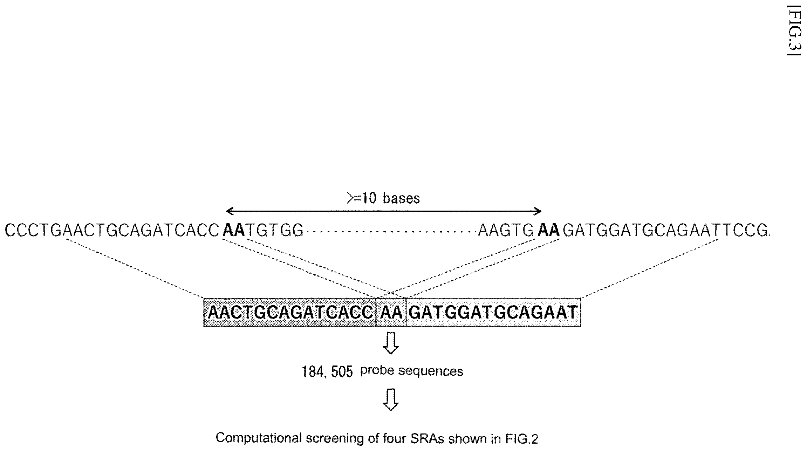

[FIG. 3] Figure 3 shows a method for constructing a probe sequence for computational APP gencDNA screening. A search for sequences was performed such that the two bases at the 3'-end in some sequences were identical to the two bases at the 5'-end in other sequences. Sixteen bases including the homologous two bases at the 3'-end were bound to sixteen bases including the homologous two bases at the 5'-end, and a pair of homologous two bases were removed to prepare a probe sequence having 30 bases. Regions of construction of the probe sequence were selected in coding regions of the APP gene and so as to separate the homologous regions away from each other by at least 10 bases. Probe sequences that can be itself mapped directly to APP cDNA were removed to obtain a final set of 184,506 probe sequences.
[Figure 4] Figure 4 is a graph of APP gencDNA compared between SAD and NCI in PRJNA574438 constructed from cf-mRNA. The results of Mann-Whitney U test analysis on APP gencDNA capable of producing Aβ are presented. The p value is 0.0000318.
[Figure 5] Figure 5 is a graph of APP gencDNA compared between SAD and NCI in PRJNA574438 constructed from cf-mRNA. The results of Mann-Whitney U test analysis on delex8 are presented. The p value is 0.0127.
[Figure 6] Figure 6 is a graph of an examined correlation between the number of normalized reads capable of producing Aβ and delex8. R² = 0.2085.
[Figure 7] Figure 7 is a graph of APP gencDNA compared between SAD and NCI in PRJNA574438 constructed from cf-mRNA. The results of Mann-Whitney U test analysis on APP gencDNA capable of producing Aβ except for reads with proseqff178928 are presented. p value: 0.00207.
[Figure 8] Figure 8 is a graph of APP gencDNA compared between SAD and NCI in PRJNA574438 constructed from cf-mRNA. The results of Mann-Whitney U test analysis on reads with only proseqff178928 are presented. p value: 0.0000153.
[Figure 9] Figure 9 is a graph of APP gencDNA compared between SAD and NCI in PRJNA574438 constructed from cf-mRNA. The results of Mann-Whitney U test analysis on reads with only proseqff178928 normalized with DRA are presented. p value: 0.00000148.
[Figure 10] Figure 10 shows representative read sequences having a site of connection in exon which is detected in blood plasma.
[Figure 11] Figure 11 is a table showing probe sequences detected in PRJNA493258 to SRR7905478 and SRR7905479.
[Figure 12] Figure 12 is a table related to detection of APP gencDNA in published NGS data (SRA).
[Figure 13] Figure 13 is a table showing top ten probe sequences in terms of a read count in PRJNA574438.
[Figure 14] Figure 14 is a table related to probe sequences of APP gene DNA in blood plasma CNA in Example 1, which are detected with an Illumina sequence.
[Figure 15] Figure 15 is a table showing the results of Example 2.
[Figure 16] Figure 16 shows amplification curves showing the results of Example 3.
[Figure 17] Figure 17 is a graph showing the results of Example 4.
[Figure 18] Figure 18 is a graph showing the results of Example 5.
[Figure 19] Figure 19 is a graph showing the results of Example 6.

### Description of Embodiment

### <<Diagnosis marker detection method>>

A diagnosis marker detection method of the present invention comprises detecting a disease-related gene from circulating nucleic acids (CNAs) in peripheral blood isolated from a subject.

APP mRNA reverse-transcribed, caused to undergo intramolecular recombination and randomly inserted into genomic DNA (referred to as genomic cDNA or "gencDNA") in brain neurons after death of an Alzheimer's disease patient has been reported (see Somatic APP gene recombination in Alzheimer's disease and normal neurons. Lee MH, Siddoway B, Kaeser GE, Segota I, Rivera R, Romanow WJ, Liu CS, Park C, Kennedy G, Long T, Chun J. Nature. 2018 Nov; 563(7733): 639-645.).

On the basis of the findings described above, the present inventors have found fragmented DNA derived from gencDNA of APP in cell-free circulating nucleic acids in peripheral blood of an Alzheimer's disease patient.

Conventional detection of APP has been performed exclusively in the brain or with cerebral fluid but it has not been recognized as preferable from the viewpoint of invasiveness. The diagnosis of Alzheimer's disease using peripheral blood, which has been discovered by the present inventors, is convenient and noninvasive. In this concept, the present invention can also be applied to other diseases. Examples of the disease include neurological diseases such as Alzheimer's disease, Parkinson's disease, amyotrophic lateral sclerosis (ALS), Huntington's disease, prion disease, cerebral edema, brain ischemia, brain infarction, brain tumors, demyelinating disease, epilepsy, neuropathic pain, migraine, manic depressive psychosis, major depressive disorders and schizophrenia. In addition, the present invention is applicable to any diseases that develop in a tissue-specific manner. Examples thereof include tissue-specific cancers such as brain tumors, bladder cancer, lung cancer, bowel cancer, stomach cancer, breast cancer, prostate cancer and thyroid cancer.

The disease-related gene is not limited as long as it is expressed in a disease-specific manner, and examples thereof include neurological disease-related genes, and cancer genes.

Examples of the neurological disease-related gene include APP, Snca, Uchl1, Apoe, Park7, Apbb1, Bcl2, Ube2l1, Ubqln1, Bax, Cdk5, Ubb, Als2, Gtf2a1, Bace1, Psen1, Ide, Ccs, Sod1, Gpx1, Hsp90AA1, Hsp90AB1, Hsp4 (Hsp70), DNAJB1 (Hsp40), Stip1, Hsf1, and Mapt (tau).

Examples of the cancer gene include a group of genes encoding a proliferative factor such as sis; a group of genes encoding receptor tyrosine kinase such as erbB, fms or ret; a group of genes encoding non-receptor tyrosine kinase such as fes; a group of genes encoding a GTP/GDP-binding protein such as ras; a group of genes encoding serine/threonine kinase such as src, mos or raf; a group of genes encoding an intranuclear protein such as myc, myb, fos, jun or erbA; a group of genes encoding a signaling adapter molecule such as crk; and fusion genes such as Bcr-Abl.

Further, examples of the cancer gene include Ras-MAP kinase pathway-related genes such as Shc, Grb2, Sos, MEK, Rho and Rac genes; phospholipase C gamma-protein kinase C pathway-related genes such as PLCγ and PKC; PI3K-Akt pathway-related genes such as PI3K, Akt and Bad; JAK-STAT pathway-related genes such as JAK and STAT; and GAP pathway-related genes such as GAP, p180 and p62.

Hereinafter, as an example, an Alzheimer's disease diagnosis marker detection method will be described.

### <Alzheimer's disease diagnosis marker detection method>

In this embodiment, the present invention provides a diagnosis marker detection method comprising detecting an amyloid precursor protein (APP) gene from cell-free circulating nucleic acids in peripheral blood isolated from a subject.

The peripheral blood is acquired by a conventional method, and the cell-free circulating nucleic acids are extracted by a conventional method.

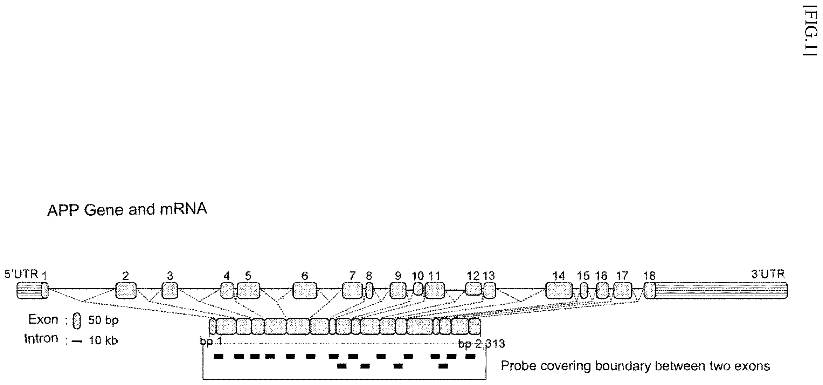

As shown in Figure 1, the APP gene has 18 exons, and a mRNA precursor obtained by transcription is spliced into mRNA. Plural splicing variants have been reported, and APP695 mRNA free of exon 7 and exon 8 is specifically expressed in brain neurons (Neuronal ELAVL proteins utilize AUF-1 as a co-partner to induce neuron-specific alternative splicing of APP. Fragkouli A, Koukouraki P, Vlachos IS, Paraskevopoulou MD, Hatzigeorgiou AG, Doxakis E. Sci Rep. 2017 Mar 14; 7: 44507). Mechanisms of gencDNA formation have not been clarified, but it is thought that mRNA of the splicing variant is reverse-transcribed, turns into cDNA, undergoes intramolecular recombination, and is randomly inserted into genomic DNA. The inserted cDNA is called gencDNA.

The present inventors have found that diagnosis of Alzheimer's disease can be made by detecting gencDNA or a transcription product from the gencDNA released into peripheral blood upon cell death. The detection method is preferably detection of a site of connection or a site of homologous recombination between exons of the APP gene as a disease-related gene for detecting gencDNA formed with a splicing variant being reverse-transcribed and undergoing intermolecular recombination, or a transcript thereof.

Specific examples thereof include amplifying a site of connection between exons by PCR using a forward primer annealed to a specific exon and a reverse primer annealed to another exon, and/or labeling the relevant site of connection using a probe hybridizable to a region containing the site of connection between exons or a site of homologous recombination. A combination of various primers which can anneal, respectively, to exons 1 to 18 so that it is possible to perform detection regardless of which exons between which a fragment is caused to undergo intramolecular recombination. It is preferable to detect a site of connection between exon 7 and exon 9 among others. Alternatively, it is preferable to detect a site of connection between exon 14 and exon 16. Note that this site of connection is identical to L-APP mRNA which is mRNA of APP lacking exon 15 and which is expressed in leukocytes and glial cells.

In addition, the detection method is preferably detection of a site of homologous recombination between exons of the disease-related gene.

Alternatively, mRNA of a gene related to Alzheimer's disease, for example, a NUMB gene or a PROK 2 gene may be detected because the mRNA is also released from cells of an injured brain.

Further, from the viewpoint of diagnosing the degree of progression or severity of Alzheimer's disease, the number of copies of a disease-related gene detected is preferably quantitated. Specific examples of the quantitation method include two types of method: a next-generation sequencer (NGS) method and a real-time PCR (RT-PCR) method.

### [Next-generation sequencer (NGS)]

In the next-generation sequencer, the analyzed DNA fragment is called a read, and a product of the number of reads and the number of bases determined per read (read length) gives output data.

In this embodiment, the quantitation is preferably performed by amplifying a region containing a site of connection between exons by singleplex PCR or multiplex PCR; analyzing the nucleotide sequence of the purification product with a next-generation sequencer (NGS); and counting the number of reads in the region containing the site of connection, a homologous recombination region, or an Alzheimer's disease-related gene, for example, a NUMB gene or PROK 2 gene. The multiplex PCR is a method in which plural gene regions are simultaneously amplified by simultaneously using a plurality of primer pairs for one PCR reaction system. Specifically, the quantitation is preferably performed by, using a reverse transcript of CNA purified from blood plasma, amplifying a region containing the site of connection or the site of homologous recombination between exons by singleplex PCR or multiplex PCR; analyzing the nucleotide sequence of an amplification product thereof with a next-generation sequencer (NGS); and counting the number of reads in the region containing the site of connection or the site of homologous recombination.

From the viewpoint of quantitation, standardization (normalization) is preferably performed by, during PCR of a region containing a site of connection between exons, amplifying an internal standard in the same PCR tube; and comparing the number of reads in the internal standard with the number of reads derived from the site of connection between exons, the site of homologous recombination or the Alzheimer's disease-related gene.

In diagnosis of Alzheimer's disease, the internal standard preferably contains exon 8 of the APP gene. In addition, the internal standard may contain an exon region of the HLA-DRA gene.

Further, a cut-off value may be determined by an average value and a standard deviation in a non-disease group standardized using the internal standard.

### [Real-time PCR (RT-PCR)]

In real-time PCR, the amplification product formed can be monitored by detecting the fluorescence intensity using an intercalator method, a probe method, a cycling probe method or the like.

In the present embodiment, a region containing a site of connection between exons is preferably quantitated by a singleplex or multiplex real-time PCR (RT-PCR) method. In the multiplex PCR, at least a part of a combination of various primers annealed, respectively, to the sites of connection or sites of homologous recombination may be used in one PCR reaction system.

Specifically, a region containing the site of connection or the site of homologous recombination between exons, or the Alzheimer's disease-related gene is preferably quantitated by a singleplex or multiplex one-step real-time PCR (RT-PCR) method comprising a reverse transcription step.

From the quantitation, standardization is preferably performed by using a fluorescent dye-labeled probe specific to the site of connection between exons and an internal standard-specific fluorescent dye-labeled probe labeled with a fluorescent dye different from a fluorescent dye of the fluorescent dye-labeled probe specific to the site of connection between exons; and comparing the fluorescence intensities of the probes.

Examples of the quantitation method include an absolute quantitation method in which the actual number of copies of a target and a comparative quantitation method in which a relative value between samples is determined. Which of the methods is used depends on the nature of data to be acquired.

In diagnosis of Alzheimer's disease, the internal standard preferably contains exon 8 of the APP gene. In addition, the internal standard may contain an exon region of the HLA-DRA gene. Further, a cut-off value may be determined by an average value and a standard deviation in a non-disease group standardized using the internal standard.

Standardization is preferably performed by, during PCR of a region containing a site of connection between exons, amplifying an internal standard in the same PCR tube; and comparing the number of reads in the internal standard with the number of reads in the site of connection or the site of homologous recombination between exons, or the Alzheimer's disease-related gene; or standardization is performed by, during RT-qPCR, using a fluorescent dye-labeled probe specific to the site of connection or the site of homologous recombination between exons or the Alzheimer's disease-related gene, and an internal standard-specific fluorescent dye-labeled probe labeled with a fluorescent dye different from a fluorescent dye of the fluorescent dye-labeled probe specific to the site of connection or the site of homologous recombination between exons or the Alzheimer's disease-related gene; and comparing the fluorescent intensities of the probes.

The diagnosis marker detection method according to this embodiment is a technique for detecting fragmented DNA or mRNA released into blood upon cell death and enables sensitive detection of a diagnosis marker, because it is possible to pass through a nucleic acid amplification reaction.

### <<Diagnostic kit>>

The diagnostic kit of the present invention is a diagnostic kit for detecting a disease-related gene from CNAs in peripheral blood isolated from a subject, the diagnostic kit comprising a primer set for amplifying the disease-related gene or a transcription product therefrom, and/or a probe that binds to the disease-related gene or an amplification product thereof. The diagnostic kit of the present invention is suitably used in <<Diagnosis marker detection method>> above, and has the configuration described in <<Diagnosis marker detection method>>.

In the case of a diagnostic kit for Alzheimer's disease, the target disease-related gene is preferably an APP gene, a NUMB gene or a PROK 2 gene, and it is more preferable that the primer set and/or the probe detect a site of connection or a site of homologous recombination between exons of the APP gene, or detect mRNA derived from the NUMB gene or mRNA derived from the PROK 2 gene. Specific examples of the primer set and probe include primer sets and probes substantially identical to those represented by nucleotide sequences set forth as SEQ ID NOS: 1 to 6 and 15 to 52.

The term "substantially identical" means that each of the primer set and the probe has an identity of at least 80% (for example, 85%, 90%, 95%, 96%, 97%, 98%, 99% and 100%) with a nucleotide sequence set forth as a relevant sequence number so that it can anneal or hybridize to target nucleic acids.

It is preferable to target exon 8 of the APP gene or the exon part of the HLA-DRA gene as an internal standard.

Further, the kit may include, in addition to the primer set and/or probe, a kit for separating peripheral blood from blood and/or a kit for extracting CNA from peripheral blood.

### Examples

Hereinafter, the present invention will be described by way of Examples, which should not be construed as limiting the present invention.

### [Computational screening with probe sequences of published Pacbio sequence data]

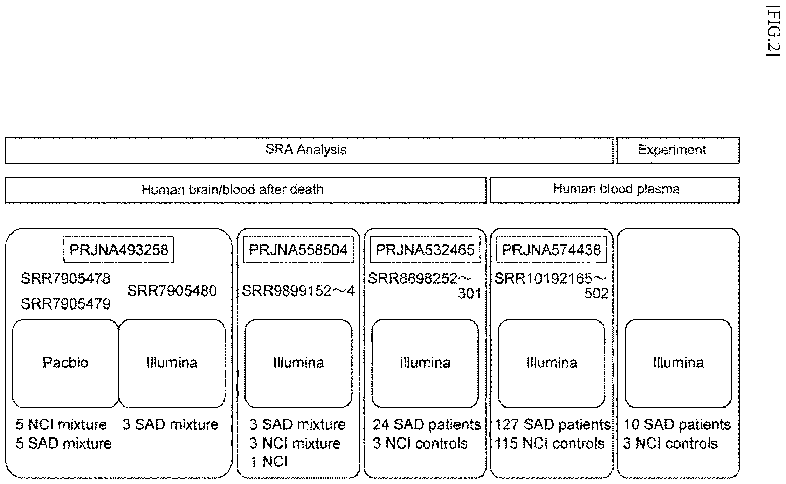

For confirming the presence of APP gencDNA and understanding the general scope thereof, probe sequences were designed on the basis of the APP mRNA sequence, and published sequence data (sequence read archive, SRA) was screened with a computer (see Figure 2). The reported APP gencDNA was formed by homologous recombination in exon with a homologous region of two bases. Therefore, the probe sequence was prepared by combining 14 bases upstream of a homologous region of two bases, a homologous sequence of two bases, and 14 bases downstream of the homologous region. A total of 184,506 probe sequences were obtained (see Figure 3). Next, the constructed probe sequences were used in computational screening of SRR7905478 and SRR7905479 of BioProject PRJNA493258 which had been obtained by Pacbio-sequencing of amplicons of nestedPCR of APP of the human brain after death.

The number of positive reads in the probe sequence of SRR7905479 in the SAD case was 190,934 among a total of 254,351 reads. For SRR7905478 in the NCI case, the number of positive reads was 82,346 among a total of 360,290 reads.

These SRAs were constructed after nestedPCR of APP amplified between exons 1 and 18, and therefore were not compatible with general normalization using a housekeeping gene, and these reads were normalized by the total number of reads. Results of 0.751 for the SAD case and 0.229 for the NCI case were obtained. These results show that recombination in APP exon occurs more frequently in the SAD case than in the NCI case.

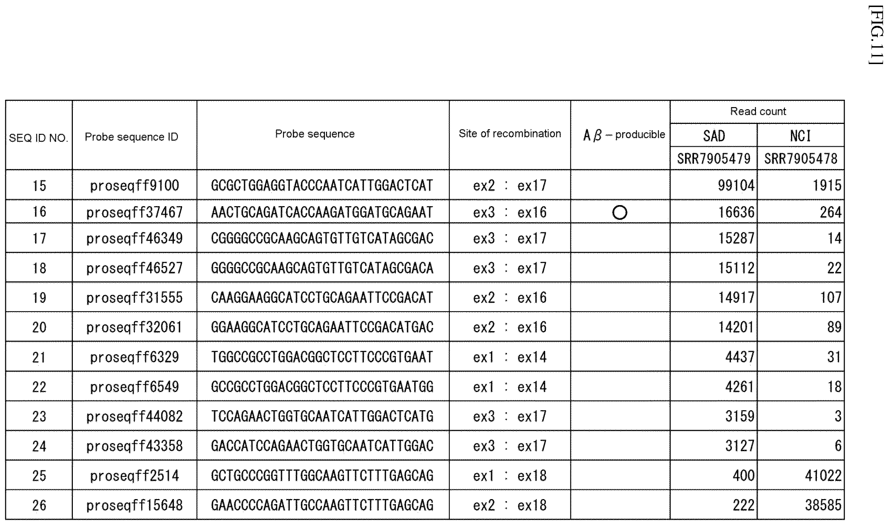

Comparison between the positive probe sequences of SAD and NCI showed that for a total of 38 probe sequences in each of these two runs, 26 probe sequences were positive in SRR7905479 (SAD) and 25 probes were positive in SR7905478 (NCI). Figure 11 shows SADs with 200 or more reads. Four of 38 probe sequences had no frame shift at a site of connection, and were thought to be potentially capable of producing Aβ.

The proseqff37467 sequence having the largest number of Aβ producing reads was also positive in the NCI group, but had a read count smaller than that in the SAD group (NCI: 264, SAD: 16,636). On the other hand, APP mRNA lacking exon 8 was specific to neurons, and SRR7905478 and SRR7905479 were constructed from genomic DNA without reverse transcription. Therefore, a connection sequence between exons 7 and 9 (delex 8) of genomic DNA of the brain after death may be derived from APP gencDNA. The number of reads in the connection sequence was 816 (163 per case) for SRR7905478 (NCI) and 385 (77 per case) for SRR7905479 (SAD). When normalization was performed using the number of reads in each of these connection sequences, the exponent was 0.32 for NCI and 43.2 for SAD. Further, three additional Aβ producing probe sequences were observed in the SAD group, but were not observed in the NCI group. These results show that the proportion of Aβ producing reads in the recombinant in APP exon is much higher in the SAD group than in the NCI group of SRA PRJNA493258 including SRR7905478 and SRR7905479.

### [Computational screening with probe sequence in published Illumina sequencing data]

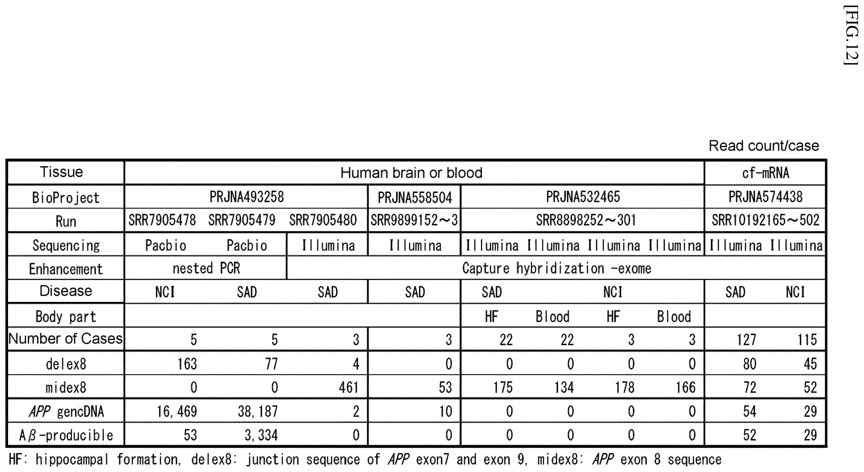

Since the constructed probe sequences can be used in the APP gencDNA screening, other SRAs were also analyzed (see Figure 2). In the two runs of SRR7905480 of BioProject PRJNA493258 and SRR9899152-3 of BioProjectPRJNA558504, there were one and two positive probe sequences, respectively, but the numbers of reads were as small as 5 (2/case) and 29 (10/case), respectively (see Figure 12).

In BioProject PRJNA532465, a very small number of probe sequences (0.14 reads per case) were observed in the hippocampal formation (HF) for the SAD case, but positive probe sequences were not observed in blood for the SAD case or HF and blood for the NCI case. On the other hand, the average number of reads in the APP exon 8 (midex8) was 134 to 178 (see Figure 12).

These three SRAs were constructed from the brain and blood after death by capture hybridization and Illumina sequence unlike SRR7905478 and SRR7905479 obtained from nestedPCR amplicons of APP.

### [Comparison of number of APP gencDNA reads in SRA from blood plasma cf-mRNA]

In contrast, PRJNA574438 constructed from cf-mRNA (cell-free mRNA) as opposed to the brain after death had 331 positive probe sequences, two of which were identical to those detected in BioProject SRR7905480 and PRJNA558504. However, there was no common positive probe sequence between SRR7905478 and SRR7905479 in which many probe sequence reads were detected. The number of positive probe sequences in PRJNA574438 was 125 per 127 cases for SAD, and 96 per 116 cases for NCI. There was a significant difference in the average number of positive probe sequences, which was 4.5 for SAD and 2.4 for NCI (p value in Welch two sample t-test: 1.74E-4). The average total number of APP gencDNA reads was 54/case for SAD and 29/case for NCI. When normalization by the number of reads in midex8 was performed, there was a significant difference in the number of APP gencDNA reads between SAD and NCI. The p value in the Mann-Whitney U test was 1.88E-5. For the NCI case in which the number of APP gencDNA reads, there were six outliers. Specifically, the MMSE score was 30 in three cases, 29 in one case, and unavailable in two cases.

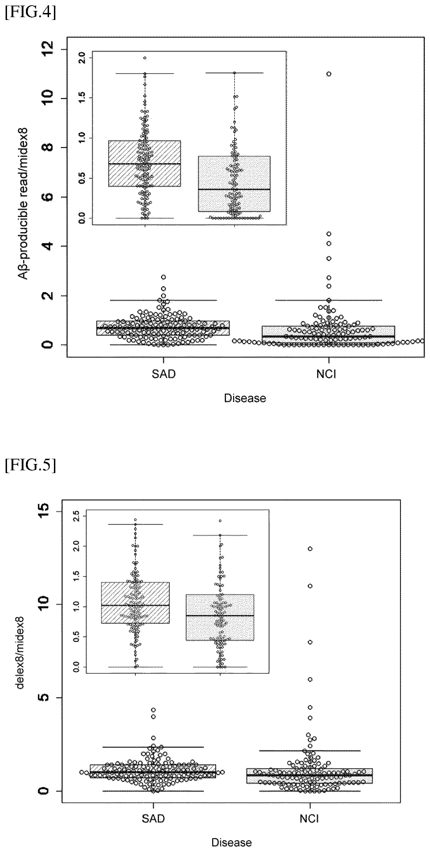

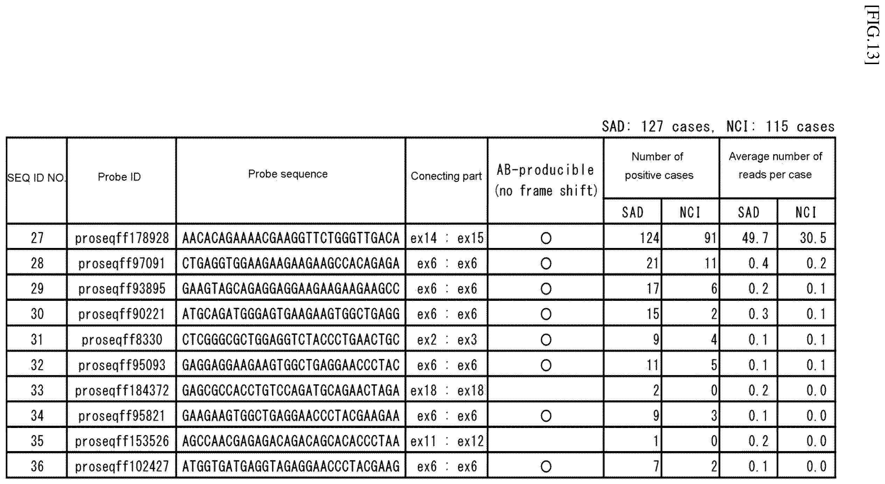

For Aβ formation, a frame shift did not occur in 202 probe sequences, among 301 probe sequences. The reads thereof are considered to be capable of forming Aβ, but except for proseqff178928, the number of reads was small. Figure 13 shows the top ten numbers of reads capable of forming Aβ. With regard to reads capable of forming Aβ, the average number of reads was 52/case for SAD, and 29/case for NCI. These values were the same as the above-described numbers of APP gencDNA reads. Even when normalization was performed by midex8, there was a significant difference between SAD and NCI (p value in Mann-Whitney U test: 2.9E-5, see Figure 4), and there was a high correlation between the normalized total number of reads and the number of Aβ producing reads (r² = 0.982). In the NCI case, there were six outliers, and they were consistent with the above-described case of outliers observed for the normalized total APP gencDNA read count.

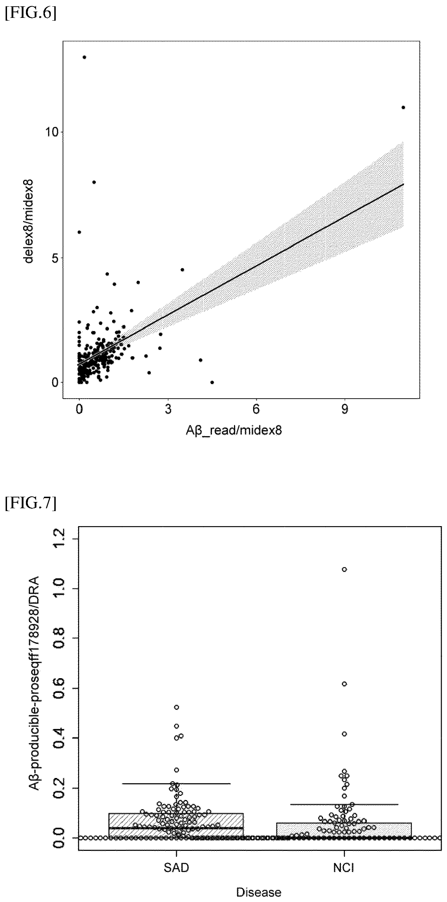

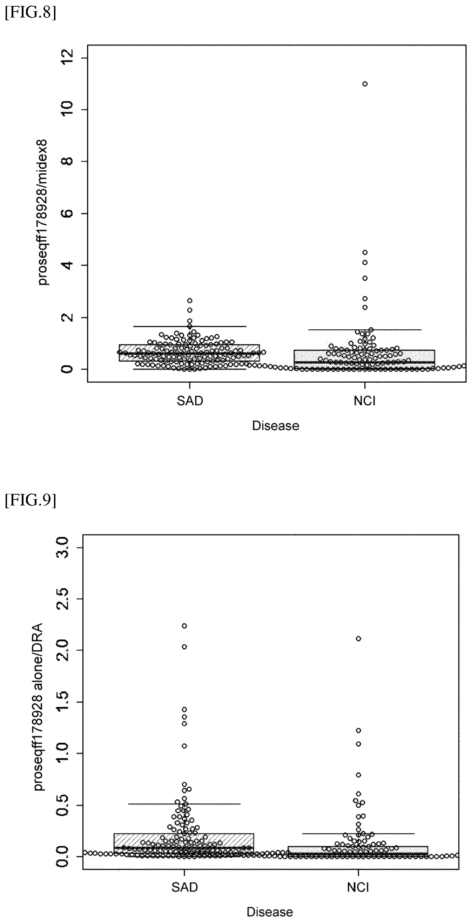

The most frequent probe sequence was proseqff178928, and accounted for about 89% of probe sequence positive reads, and the sequence thereof was identical to the sequence of the site of connection between exons 14 and 16 in APP mRNA lacking exon 15, which had been termed L-APP mRNA. L-APP mRNA is expressed in leucocytes and astrocytic cells. Therefore, with consideration given to the possibility that L-APP mRNA had been released from those cells, reads were examined with only proseqff178928. In the case where proseqff17892 was excluded from the Mann-Whitney U test, the p value was 2.07E-2 (see Figure 7). The p value of proseqff178928 alone was 1.53E-5 (see Figure 8). That is, the p value of only the probe sequence proseqff178928 was more significant over the p values of all probe sequence positive reads (p value: 3.18E-5).

### [Example 1]

### [Analysis of nucleotide sequence of circulating nucleic acids]

For confirming the presence of a transcription product from APP gencDNA in blood plasma, NGS analysis was performed with a blood plasma sample. Circulating nucleic acids were extracted from 1 to 1.5 mL of blood plasma using QIAamp Circulating Nucleic Acid Kit (QIAGEN), and eluted to 17 µL. Using 15 µL of the eluate, ProtoScript II First Strand cDNA Synthesis Kit and NEBNext Ultra II Non-Directional RNA Second Strand Synthesis Module (NEB), double-stranded cDNA was synthesized. The cDNA solution also contained cfDNA because a procedure for removing cfDNA was not carried out. The cDNA solution was purified using 1.2 times its volume of AMPure XP beads (Beckman Coulter, Inc.), dA tailing was performed using NEBNext Ultra II End Repair/dA-Tailing Module (NEB), and adaptor ligation was then performed using NEBNext Ultra II DNA Library Prep Kit for Illumina. The reaction mixture was purified using 1.2 times its volume of AMPure XP beads, and eluted with 32 µL 0.1 x TE. The cfDNA and cDNA were concentrated by capture hybridization using a xGen capture probe (Integrated DNA Technologies, Inc.), and then PCR amplification using KAPA HiFi HotStart ReadyMix (Kapa Biosystems, Inc.). Next, Illumina sequencing was performed using MiSeq Reagent Kit v2 (300 cycles) and MiSeq Sequencer (Illumina, Inc.).

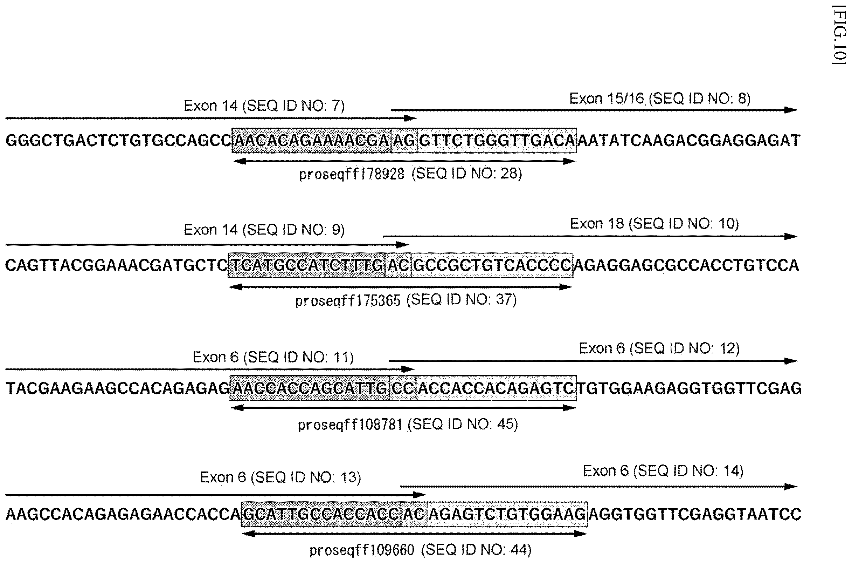

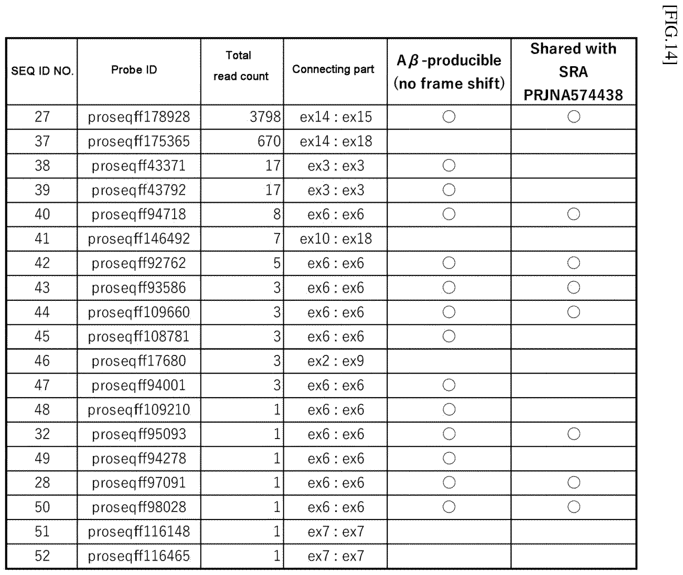

A total of 19 probe sequences were detected in the blood plasma sample (see Figure 14). Those sequences were detected in 7 out of 10 SAD patients, and 2 out of 3 controls. Read sequences containing probes detected in two sequencing runs are shown in Figure 10. Interestingly, 8 probe sequences were identical to those detected in SRA PRJNA574438 constructed from blood plasma cf-mRNA. Further, the blood sample and PRJNA574438 were identical in the probe sequence having the largest number of reads.

### [Comparison of number of reads in sequence of junction between exon 7 and exon 9 of APP]

In a probe sequence constructed on the assumption of the homologous recombination of two nucleotides, a connection part cannot be detected. Since APP gencDNA is considered to be formed by reverse transcription of mRNA, that is, APP mRNA expressed in neurons lacks exon 8, the APP gencDNA includes one having a distinct junction sequence between exons 7 and 9 of APP. Thus, computational screening of SRA was performed using delex8 which is a nucleotide sequence of a site of connection between exon 7 and exon 9. However, cDNA synthesized from APP mRNA derived from neurons has the same connection part as delex8, and therefore unlike the probe sequences prepared as described above, delex8 is specific to neurons as opposed to being specific to APP gencDNA. The results are shown in Figure 12. A box blot of delex8 normalized by midex8 of PRJNA574438 constructed from cf-mRNA is shown in Figure 5. In the Mann-Whitney U test, a significant difference was observed between SAD and NCI, and the p value was 0.0127.

### [Example 2]

From 1 mL of blood plasma from each of a control having no cognitive dysfunction and an Alzheimer's disease (AD) patient, CNA was extracted using QIAamp Circulating Nucleic Acid Kit (QIAGEN N.V.).

As shown in Figure 1, the APP gene has 18 exons, and a mRNA precursor obtained by transcription is spliced into mRNA.

PCR was performed using the extracted CNA and the following primers.
Forward primer: site of connection between exon 1 and exon 2
   GGCACTGCTCCTGCTGG (SEQ ID NO: 1)
Reverse primer: site of connection between exon 17 and exon 18
   CAGGTGGCGCTCCTCTG (SEQ ID NO: 2)

PCR cycles are as follows.
98°C 30 sec: 1 cycle
95°C 15 sec, 60°C 30 sec, 72°C 30 sec: 40 cycles
4°C: hold

Thereafter, a library was prepared, and nanopore sequencing was performed.

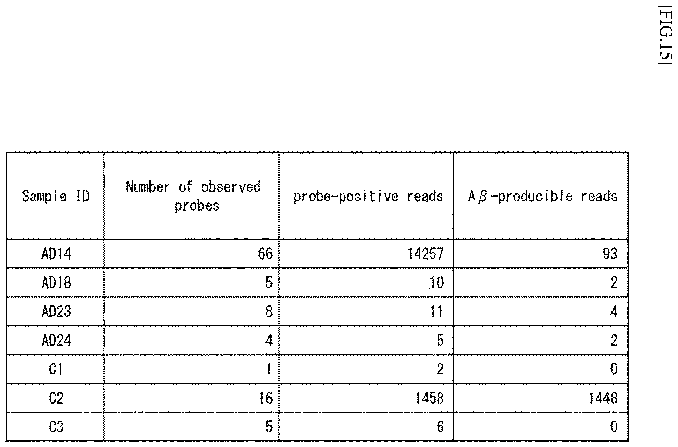

The results are shown in Figure 15. The sample derived from the Alzheimer's disease patient was confirmed to be significantly different in the number of positive reads in the probe from the control sample.

### [Example 3]

Using CNA prepared in the same manner as in Example 1, one-step RT-PCR was performed. A TaqMan one-step RT-PCR reagent from Thermo Fisher Scientific was used.

The following primers and probes were used.
Forward primer: exon 7 CACCACCACCACCACAGAG (SEQ ID NO: 3)
Reverse primer: exon 9 GATACTTGTCAACGGCATCAGG (SEQ ID NO: 4)
Fam-labeled probe: GGCAGCGCCATTCCTACAACAG (detecting a site of connection between exon 7 and exon 9 (delex8); SEQ ID NO: 5)
Hex-labeled probe: CAAGACTACCCAGGAACCTCTTGCC (detecting exon 8 (midex8); SEQ ID NO: 6)

PCR cycles are as follows.
50°C 5 min: 1 cycle
95°C 20 sec: 1 cycle
95°C 15 sec, 60°C 60 sec: 40 cycles

A probe labeled with HEX, a fluorescent dye, was used as a probe for the internal standard, and a probe labeled with FAM was used as a probe specific to a site of connection between exons.

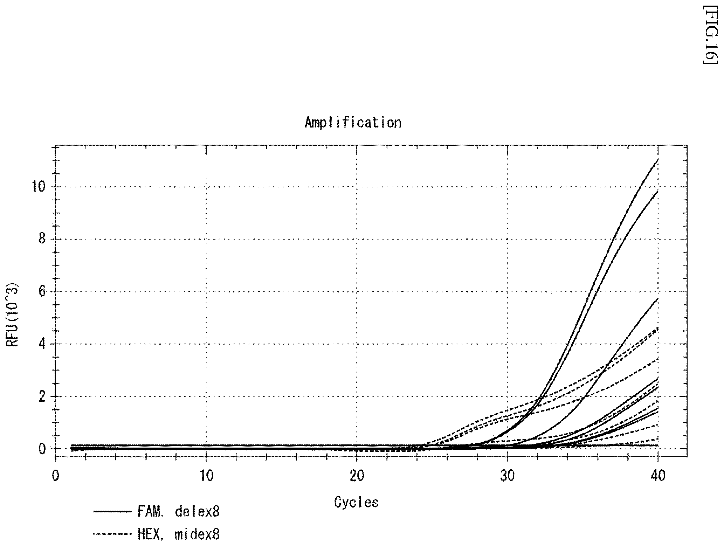

The results are shown in Figure 16. It was demonstrated that delex8 derived from neurons and midex8 as the internal standard were definitely detected even in one-step RT-PCR.

### [Example 4]

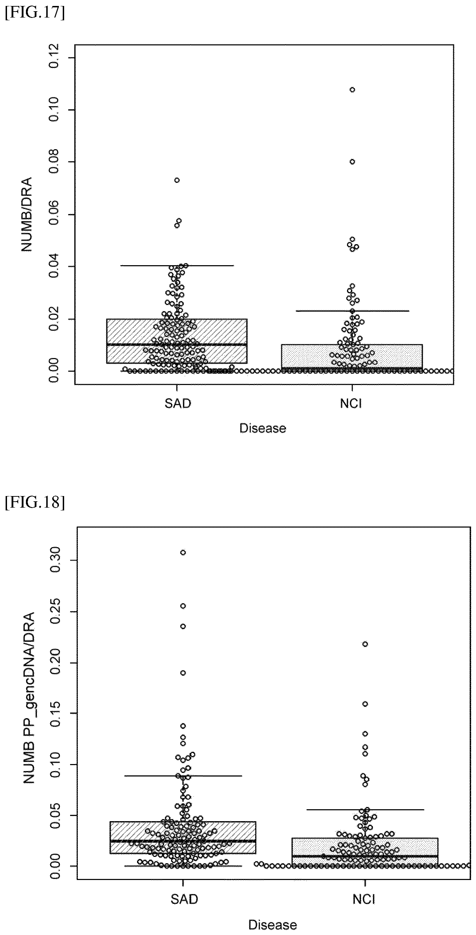

The nucleotide sequence of an exon of NUMB:
AATCCTCAGACGCCTCACTTAGGACAAGCT (SEQ ID NO: 53) was used on the published SRA of PRJNA574438 to examine the number of reads containing this sequence, which was compared between SAD and NCI. For standardization, the nucleotide sequence of the HLA-DRA gene:
GGCAAAGAAGGAGACGGTCTGGCGGCTTGA (SEQ ID NO: 54) was used. In the Mann-Whitney U test, a P value of 0.000000305 was obtained. The results are shown in Figure 17.

### [Example 5]

The numbers of reads in APP gencDNA standardized by HLA-DRA and NUMB were combined, and similarly compared. In Mann-Whitney U test, a P value of 0.000000165 was obtained. The P value was smaller than that is the case of NUMB alone. The results are shown in Figure 18.

### [Example 6]

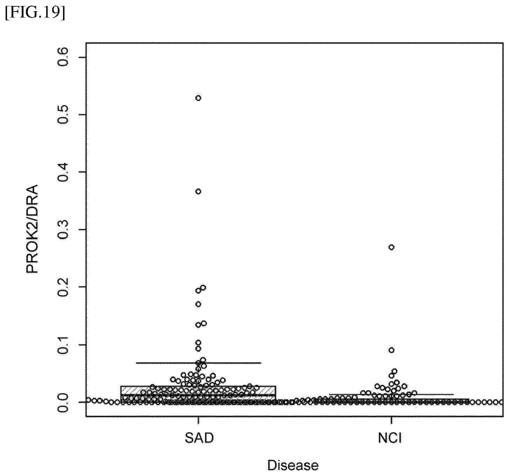

The nucleotide sequence of an exon of a variant of the PROK 2 gene: AATGGAAGGCAGGAAAGAAGAAAGAGGAAG (SEQ ID NO: 55) was used on the published SRA of PRJNA574438 to examine the number of reads containing this sequence, which was compared between SAD and NCI. For standardization, the nucleotide sequence of the HLA-DRA gene:
GGCAAAGAAGGAGACGGTCTGGCGGCTTGA (SEQ ID NO: 54) was used. In the Mann-Whitney U test, a P value of 0.000000000012 was obtained. The results are shown in Figure 19.

According to the present invention, it is possible to provide a diagnosis marker detection method and a diagnostic kit which enable convenient, sensitive, rapid and noninvasive diagnosis of Alzheimer's disease.

## Claims

1. A diagnosis marker detection method comprising detecting a disease-related gene from circulating nucleic acids (CNAs) in peripheral blood isolated from a subject.

2. The diagnosis marker detection method according to claim 1, wherein the disease is Alzheimer's disease.

3. The diagnosis marker detection method according to claim 1 or 2, wherein the disease-related gene is an amyloid precursor protein (APP) gene.

4. The diagnosis marker detection method according to any one of claims 1 to 3, wherein a site of connection between exons of the disease-related gene is detected.

5. The diagnosis marker detection method according to claim 4, wherein the site of connection between exons of the disease-related gene is a site of connection between exon 7 and exon 9.

6. The diagnosis marker detection method according to claim 4, wherein the site of connection between exons of the disease-related gene is a site of connection between exon 14 and exon 16.

7. The diagnosis marker detection method according to any one of claims 1 to 3, wherein a site of homologous recombination between exons of the disease-related gene is detected.

8. The diagnosis marker detection method according to claim 1 or 2, wherein the disease-related gene is a NUMB endocytic adaptor protein gene (NUMB gene) or a prokineticin 2 gene (PROK 2 gene).

9. The diagnosis marker detection method according to claim 1 or 2, wherein the disease-related gene is a combination of genes selected from the group consisting of an APP gene, a NUMB endocytic adaptor protein gene (NUMB gene) and a prokineticin 2 gene (PROK 2 gene).

10. The diagnosis marker detection method according to any one of claims 1 to 9, further comprising quantitating the number of copies of the detected disease-related gene.

11. The diagnosis marker detection method according to any one of claims 4 to 10, wherein the quantitation is performed by, using a reverse transcript of CNA purified from blood plasma, amplifying a region containing the site of connection or the site of homologous recombination between exons, or the NUMB gene or the PROK 2 gene by singleplex PCR or multiplex PCR; analyzing a nucleotide sequence of an amplification product thereof with a next-generation sequencer (NGS); and counting the number of reads in the region containing the site of connection or the site of homologous recombination between exons, or the NUMB gene or the PROK 2 gene.

12. The diagnosis marker detection method according to any one of claims 4 to 9, wherein the region containing the site of connection or the site of homologous recombination between exons, or the NUMB gene or the PROK 2 gene is quantitated by a singleplex or multiplex one-step real-time PCR (RT-PCR) method comprising a reverse transcription step.

13. The diagnosis marker detection method according to claim 11 or 12, wherein standardization is performed by, during PCR of the region containing the site of connection or the site of homologous recombination between exons, or PCR of the NUMB gene or PCR of the PROK 2 gene, amplifying an internal standard in the same PCR tube; and comparing the number of reads in the internal standard with the number of reads in the site of connection or the site of homologous recombination between exons or the NUMB gene or the PROK 2 gene; or standardization is performed by, during RT-qPCR, using a fluorescent dye-labeled probe specific to the site of connection or the site of homologous recombination between exons, or the NUMB gene or the PROK 2 gene, and an internal standard-specific fluorescent dye-labeled probe labeled with a fluorescent dye different from a fluorescent dye of the fluorescent dye-labeled probe specific to the site of connection or the site of homologous recombination between exons, the NUMB gene or the PROK 2 gene; and comparing fluorescence intensities of the probes.

14. The diagnosis marker detection method according to claim 13, wherein the internal standard contains exon 8 of an APP gene.

15. The diagnosis marker detection method according to claim 14, wherein the internal standard contains an exon region of an HLA-DRA gene.

16. A diagnostic kit for detecting a disease-related gene from circulating nucleic acids (CNAs) in peripheral blood isolated from a subject, the kit comprising:
a primer set for amplifying the disease-related gene, and/or a probe that binds to the disease-related gene or an amplification product thereof.

17. The diagnostic kit according to claim 16, wherein the disease is Alzheimer's disease.

18. The diagnostic kit according to claim 16 or 17, wherein the disease-related gene is at least one selected from the group consisting of an amyloid precursor protein (APP) gene, a NUMB endocytic adaptor protein gene and a PRPK gene.

19. The diagnostic kit according to any one of claims 16 to 18, wherein a site of connection or site of homologous recombination between exons of the disease-related gene or an exon of the gene is detected.
